(19) **Europäisches Patentamt / European Patent Office / Office européen des brevets**

(11) **EP 4 715 757 A1**

(12) # EUROPEAN PATENT APPLICATION

(43) Date of publication:
25.03.2026 Bulletin 2026/13

(21) Application number: 24202134.3

(22) Date of filing: 24.09.2024

(51) International Patent Classification (IPC):
**G06V 10/80** $^{(2022.01)}$ **G06T 7/00** $^{(2017.01)}$

(52) Cooperative Patent Classification (CPC):
**G06V 10/814; G06T 7/0012;** G06T 2207/10081;
G06T 2207/20076; G06T 2207/20081;
G06T 2207/20084; G06V 2201/03

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Siemens Healthineers AG**
**91301 Forchheim (DE)**

(72) Inventors:
• **Teichmann, Marvin**
**91054 Erlangen (DE)**
• **Datar, Manasi**
**400067 Mumbai (IN)**
• **Ghesu, Florin-Cristian**
**91083 Baiersdorf (DE)**
• **Kratzke, Lisa**
**91054 Erlangen (DE)**
• **Vega, Fernando**
**91056 Erlangen (DE)**

(74) Representative: **Siemens Healthineers Patent Attorneys**
**Postfach 22 16 34**
**80506 München (DE)**

(54) **A COMPUTER IMPLEMENTED METHOD FOR DETECTING AN OUT-OF-DISTRIBUTION CASE, A COMPUTER IMPLEMENTED METHOD FOR TRAINING AN EPISTEMIC BAYESIAN UNCERTAINTY MODEL, A DATA PROCESSING DEVICE, AN IMAGING SYSTEM, A COMPUTER PROGRAM PRODUCT AND A COMPUTER READABLE MEDIUM**

(57) The invention relates to a computer-implemented method for detecting an out-of-distribution case, comprising: receiving medical imaging data (22) comprising voxels, wherein the medical imaging data (22) represent an anatomical region (16) comprising an object set comprising at least one anatomical object (14), by an input interface of a data processing device (12); applying an epistemic Bayesian uncertainty model (38) on the medical imaging (22) by the computation unit to determine an epistemic uncertainty information (52) describing an epistemic uncertainty of an assignment information (54) describing an assignment of the respective voxel to the respective anatomical object (14), applying a scoring procedure on the epistemic uncertainty information (52) to determine a scoring information (36); and providing a warning signal by an output interface if the scoring information (36) satisfies a predefined out-of-distribution condition.

FIG 1

EP 4 715 757 A1

**Description**

The present invention relates to a computer implemented method for detecting an out-of-distribution case, a computer implemented method for training an epistemic Bayesian uncertainty model, a data processing device configured to carry out a computer implemented method for determining an out-of-distribution case and/or a computer implemented method for training an epistemic Bayesian uncertainty model, an imaging system, in particular a medical imaging system comprising a data processing device, a computer program product and a computer readable medium.

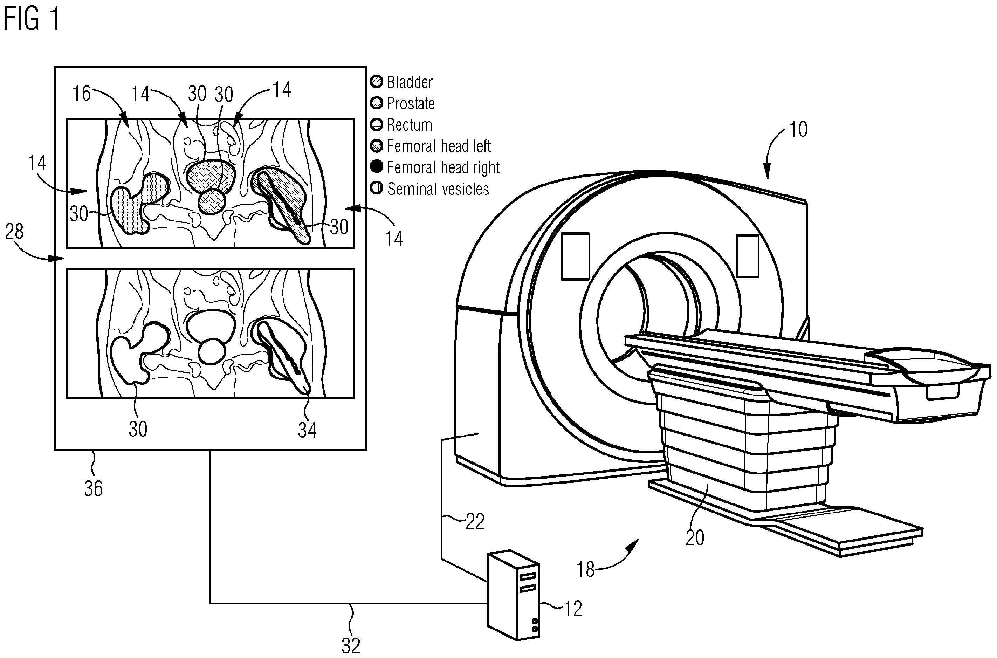

In radiotherapy, contouring is a vital process for accurately delineating target areas for treatment, including organs at risk, tumor volumes, and regions for dose computation. However, a significant challenge arises when using deep learning models for this purpose, particularly regarding out-of-distribution (OOD) data. These models, while efficient, can generate unreliable outputs when encountering scenarios not included in their training data. This unreliability poses a safety risk, especially in cases where clinicians might inadvertently use the system beyond its intended specifications, not being fully aware of its limitations. Instances such as the presence of femur implants, brachytherapy applicators, or hydrogel rectal spacers, illustrated in Figure 1, are just some examples of the challenging scenarios that can lead to inaccuracies in contouring if not included during the model's training phase. It's crucial to recognize that these are representative cases, and numerous other OOD instances could arise. The primary problem this invention aims to address is the detection of OOD data in contouring for radiotherapy. By identifying such instances, the system provides crucial warnings, alerting clinicians to potential inaccuracies and reducing the risk of using unreliable contours in treatment planning. This OOD detection is vital for ensuring safety and efficacy in radiotherapy, as it helps prevent the accidental use of the system in scenarios where its output cannot be trusted, thus mitigating the risks associated with applying AI-driven solutions outside their validated range of operation.

There are highly effective contouring model suitable to detect more than 153 organs and anatomies at risk. These contouring models have been successfully deployed on scanners, workstations and cloud in over 600 locations worldwide. Despite these advancements, traditional methods have primarily focused on improving contour accuracy, rather than explicitly quantifying or utilizing uncertainty measures like epistemic uncertainty in the contouring process.

According to the state of the art, a general uncertainty management is missing: In the context of radiotherapy, uncertainty has been acknowledged, typically in terms of anatomical variations and image quality. However, these uncertainties are often addressed through rigorous training, standardized protocols, and manual review processes, rather than through a systematic, quantifiable approach.

According to the state of the art there is a lack of epistemic uncertainty utilization. There has been a significant gap in the explicit use of epistemic uncertainty for risk mitigation in radiotherapy contouring. While some contouring models in medical imaging might implicitly account for uncertainty, they do not typically provide a clear, quantifiable measure of epistemic uncertainty that can be directly used to inform clinical decisions or flag potential out-of-distribution cases.

Recent advancements in AI have started to explore the incorporation of uncertainty measures, but these have not been widely adopted in clinical practice, especially in the specific context of radiotherapy contouring. The utilization of epistemic uncertainty, particularly for detecting out-of-distribution scenarios, represents a novel approach in this field.

The current state of the art is described in the following documents:

US 11,275,976 B2 describes medical image assessment with classification uncertainty. Medical images may be classified by receiving a first medical image. The medical image may be applied to a machine-learned classifier that may be trained on second medical images. A label of the medical image and a measure of uncertainty may be generated. The measure of uncertainty may be compared to a threshold. The first medical image and the label may be output when the measure of uncertainty is within the threshold.

US 11,185,231 B2 describes intelligent multi-scale medical image landmark detection. Intelligent multi scale image parsing determines an optimal size of each observation by an artificial agent at a given point in time while searching for anatomical landmarks.

US 10,600,185 B2 discloses automatic liver segmentation using an adversarial image-to-image network on a three-dimensional medical image of a patient.

It is an objective of the present invention to provide a method that allows a detection of an out-of-distribution case in an analysis of medical imaging data.

This objective is achieved by the subject-matter of the independent claims. Further implementations and preferred embodiments are subject-matter of the dependent claims.

This invention seeks to fill this gap by integrating epistemic uncertainty assessment into the AI tool, enhancing safety and efficacy in radiotherapy contouring.

A first aspect of the present invention relates to a computer-implemented method for detecting an out-of-distribution case in medical imaging data. The medical imaging data, which comprises voxels, is received by an input interface of a data processing device. The medical imaging data refers to digital representations of the anatomical region obtained through an imaging modality such as computed tomography (CT), magnetic resonance imaging (MRI), or positron emission tomography (PET). The medical imaging data comprise a set of two-dimensional images that are reconstructed to form a three-dimensional volume, where each volume element, or voxel, contains information about the underlying tissue properties such as density, intensity, or contrast. Therefore the medical imaging data comprise voxels representing the anatomical region. Because the at least one predefined anatomical object is located in the anatomical region, at least some of the voxels represent the at least one predefined anatomical object. The anatomical region may be a predefined region of a body. The at least one predefined anatomical object may be a specific organ located in the anatomical region. The at least one anatomical object is, for example, a specific organ for which an organ contour is to be provided so that it may be highlighted in a visualization of the anatomical region.

It may be provided that assignment information on the respective assignment of each voxel is determined, which assigns the voxels to the anatomical objects represented by the voxels. The assignment information may be generated using one of the contour determination models described at the beginning. However, the assignment may be prone to error due to epistemic uncertainty. Therefore, it is necessary to determine the epistemic uncertainty of the assignment to estimate the reliability of the assignments and to determine if an out-of-distribution case is present.

The method comprises applying an epistemic Bayesian uncertainty model on the medical imaging data to determine an epistemic uncertainty information describing the epistemic uncertainty of an assignment of each voxel to its respective anatomical object. This step is carried out by a computation unit of the data processing device. The epistemic Bayesian uncertainty model comprises a deep ensemble model with base learners, where each base learner determines a respective weak assignment information for the respective voxel in a respective forward pass. The weak assignment information may comprise a value providing an assignment of the voxel to the respective anatomical object or a value providing a probability of the assignment of the voxel to the respective anatomical object or to the respective anatomical objects.

Each base learner in the deep ensemble model is trained on a respective subset of training data and comprises Monte Carlo dropout layers. In other words, each base learner in the deep ensemble model is trained independently on a separate subset of the training data. The training data may represent different samples of the anatomical region, wherein voxels of the anatomical region belonging to the respective anatomical objects are labeled with assignment information. Because each of the subsets comprises different ones of the samples, the base learners provide different outputs for a same input. The uncertainty model is structured as a deep ensemble model consisting of a predefined number of base learners, where each base learner is trained on a respective subset of the training dataset by a machine learning algorithm to generate a weak assignment information describing an assignment of the respective voxel to the respective anatomical object in a respective forward pass. In other words, each base learner in the deep ensemble model determines predictions regarding the affiliation of each voxel in the medical imaging data to the respective predefined anatomical object.

The epistemic Bayesian uncertainty model also comprises a respective Monte Carlo dropout implementation applied on the Monte Carlo dropout layers of the base learners during each forward pass. In other words, the epistemic Bayesian uncertainty model is applied on the medical imaging data by the computation unit. Each base learner within the deep ensemble model comprises Monte Carlo dropout layers. Monte Carlo dropout is used for approximating Bayesian neural networks, which allows estimating uncertainty by averaging over multiple stochastic forward passes through a neural network. The epistemic Bayesian uncertainty model, in this context, is designed to perform a respective Monte Carlo dropout in the Monte Carlo dropout layers during each forward pass through the base learner. This means that, for each base learner in the deep ensemble model, the Monte Carlo dropout layers are activated, and random subsets of neurons are dropped out during the respective forward pass. Multiple forward passes may be performed to account for stochastic variations introduced by the Monte Carlo dropout layers. The key advantage of using Monte Carlo dropout layers within the base learners is that they enable the estimation of the epistemic uncertainty by capturing the variability across multiple stochastic forward passes. This allows for the assessment of the epistemic uncertainty related to voxel-to-anatomical object assignments in medical imaging data.

In a next step, a respective epistemic uncertainty of the affiliation of the respective voxels to the respective predefined anatomical object is determined based on a variance of the predicted weak assignments of the respective voxels. In other words, to quantify an epistemic uncertainty in the predicted affiliations of each voxel to the predefined anatomical object, the epistemic uncertainty is determined for each voxel based on the variance of the predicted weak assignments across the different base learners in the deep ensemble model. Specifically, for each voxel, the predicted weak assignments from all base learners are collected. A mean and/or standard deviation of this output set may then be determined to provide the epistemic uncertainty of the predicted assignment for each of the voxels.

In a next step, a scoring procedure is applied to the epistemic uncertainty information, resulting in scoring information. If this scoring information satisfies a predefined out-of-distribution condition related to an out-of-distribution case, a warning signal is generated by an output interface of the data processing device. In other words, the scoring procedure involves assessing the epistemic uncertainty information to generate the scoring information. This scoring information reflects the confidence or reliability of voxel-to-anatomical object assignments in medical imaging data based on the epistemic uncertainty.

The out-of-distribution case may refer to a situation where the medical imaging data, deviates significantly from the distribution of the training data used for building and training the deep ensemble model. The out-of-distribution case may occur when the medical imaging data represents the anatomical region or comprises features or patterns that are not adequately represented in the training data, leading to higher uncertainty in voxel-to-anatomical object assignments. This case may typically occur when there is an implant present in the anatomical region or when a specific anomaly exists in the anatomical region, which was not represented by any of the training data during the training process.

To prove a presence of the out-of-distribution case, the scoring information may be checked for a fulfillment of a predefined out-of-distribution condition. This out-of-distribution condition may indicate that the epistemic uncertainty of voxel-to-anatomical object assignments is significantly higher than expected, suggesting the potential out-of-distribution case. If this out-of-distribution condition is satisfied, the output interface generates a warning signal to alert the user about the potential out-of-distribution case with the medical imaging data.

The present invention has the advantage, that it allows a detection of a possible out-of-distribution case in an analysis of the medical imaging data.

Unless stated otherwise, all steps of the computer-implemented method according to the first aspect of the present invention may be performed by the data processing device. In particular, the data processing device is configured or adapted to perform the steps of the computer-implemented method. For this purpose, the data processing device may for example store a computer program comprising instructions which, when executed by the data processing device, cause the data processing device to execute the computer-implemented method.

According to a further embodiment of the present invention, the computer-implemented method comprises a determining of the assignment information that describes the assignment of the respective voxel in the medical imaging data. The assignment information is determined based on the mean of the weak assignment information provided for the respective voxel by the base learners in the deep ensemble model in the respective forward passes. Once all base learners have completed their forward passes and provided their respective weak assignment information, the epistemic Bayesian uncertainty model provides the mean of the weak assignment information for each voxel. This mean value serves as the assignment information or as the base of the assignment information, describing the assignment of the particular voxel to its corresponding anatomical object. The use of a mean-based approach to determine the assignment information allows for a more robust and reliable representation of voxel-to-anatomical object assignments, as it reduces the influence of outliers or individual base learners that might be overconfident or underconfident in their weak assignment information. The embodiment has the advantage that the assignment information as well as the epistemic uncertainty information is provided by the same model.

According to a further embodiment of the present invention, the computer-implemented method comprises a determining of the assignment information that describes the assignment of the respective voxel in the medical imaging data through an application of a predefined contouring model on the medical imaging data. This process is carried out by the computation unit and involves the assignment of the respective voxel to its corresponding anatomical object by the predefined contouring model. The contouring model, also known as segmentation or delineation model, refers to a set of rules or algorithms that define how to identify and outline the boundaries of specific anatomical objects within medical imaging data. The contouring model may be based on various techniques, such as threshold-based methods, region growing, or machine learning approaches like deep neural networks.

According to a further embodiment of the present invention, the computer-implemented method Comprises generating a visualization of a predefined region of interest of the anatomical region based on the medical imaging data. For each identified anatomical object within the predefined region of interest, a respective anatomical object contour is generated. The contour encloses a volume that represents the anatomical object in question, based on voxels assigned to the corresponding anatomical object. The predefined region of interest may be a layer through the anatomical region, therefore providing a cross section through the anatomical region and the anatomical object. In this case, the anatomical object contour may be a 2D contour delimiting a cross section area of the anatomical object. The anatomical object contour may be generated based on the voxels within assigned to the respective anatomical object that are located within the layer. The predefined region of interest may also be a predefined 3D region. This embodiment allows to visualize the individual anatomical objects within the region of interest, facilitating more precise diagnosis and treatment planning. Finally, the embodiment comprises generating review data for diagnostic. The review data comprises the visualization of the predefined region of interest, the anatomical object contour for each identified anatomical object, as well as at least one epistemic uncertainty information or the scoring information. These visualization and review features allow an interpretation of the medical imaging data.

According to a further embodiment of the present invention, the computer-implemented method comprises post-processing the respective anatomical object contour as a function of the epistemic uncertainty information of at least some of the voxels assigned to the respective anatomical object and/or as a function of predefined morphological conditions. In other words, the method comprises an application of post-processing for refining the generated anatomical object contours based on the epistemic uncertainty information or predefined morphological conditions. In this embodiment, the method processes the respective anatomical object contour as a function of epistemic uncertainty information associated with at least one of the voxels assigned to the corresponding anatomical structure. Epistemic uncertainty information allows to identify voxels or areas where the delineation between adjacent anatomical objects may be unclear or ambiguous. By incorporating this information during post-processing, the method can adjust contours to better align with actual anatomical boundaries, enhancing diagnostic accuracy and confidence. It may be possible, that voxels of an epistemic uncertainty information above a predefined threshold at a boundary of the anatomical object contour are removed. Additionally, the method post-processes the respective anatomical object contour according to predefined morphological conditions. These conditions refer to a set of rules or constraints based on known characteristics of typical anatomical structures, such as shape, size, volume, or spatial relationships between adjacent structures. By applying these morphological conditions during post-processing, the anatomical object contour may be adjust and refined to provide more accurately match real-world anatomical object shapes.

According to a further embodiment of the present invention, the scoring procedure comprises determining a respective contour uncertainty score information for each contour of every identified anatomical object based on the epistemic uncertainty information associated with the voxels within the volume representing that specific anatomical object. In addition or in an alternative, a total uncertainty score information can be determined using the epistemic uncertainty information from the voxels located within the volumes encompassing all detected anatomical objects. Determining the contour uncertainty score information for each individual contour may involve analyzing the epistemic uncertainty of the voxels within the volume corresponding to that particular anatomical object. By evaluating these uncertainties, a better understanding of potential errors or inconsistencies in the contour definition may be obtained, leading to improved overall accuracy and reliability. In addition to the contour uncertainty score information, the total uncertainty score may also be calculated by examining the epistemic uncertainty information of the voxels within all volumes representing each anatomical object. This aggregated metric provides a comprehensive overview of the overall reliability and accuracy across multiple segmented structures, allowing for more efficient quality control and assessment during medical image analysis. By considering both local contour uncertainties and global total uncertainty scores. The contour uncertainties and global total uncertainty scores may be checked against respective out-of-distribution conditions to identify the out-of-distribution case.

According to a further embodiment of the present invention, the Monte Carlo dropout layers are arranged after every ResBlock layer of the respective base learner of the deep ensemble model. This configuration enables robust regularization, which helps prevent overfitting and improves the model's ability to generalize across various anatomical structures and medical imaging data sets. In this embodiment, each base learner comprises multiple ResBlock layers, which are responsible for learning and extracting relevant features from input training data. The Monte Carlo dropout layers follow each ResBlock layer, introducing stochasticity during the training process by randomly dropping a proportion of activations in the network. This randomization technique effectively prevents over-reliance on specific connections or features, promoting more diverse and comprehensive learning. The use of Monte Carlo dropout layers after every ResBlock layer within the base learner enhances the overall robustness and accuracy of the epistemic Bayesian uncertainty model. In summary, arranging Monte Carlo dropout layers after every ResBlock layer within the base learner represents an effective strategy for improving the robustness and generalization capabilities.

According to a further embodiment of the present invention, the computer-implemented method comprises receiving request information describing the object set through a request interface of the data processing device. The request information describes the object set to be analyzed. Object sets typically include one or more anatomical objects that are of particular interest in a given medical context. By explicitly specifying the anatomical object set, users can ensure that the subsequent analysis focuses on relevant and important aspects of the medical imaging data. The request information may also comprise the region of interest for providing the visualization. The request interface allows for seamless interaction between the user and the computer-implemented method, facilitating efficient communication and reducing potential barriers to accurate diagnostic outcomes. Users can easily input their desired object sets using a graphical user interface or other suitable input methods, streamlining the overall process and ensuring that the method's output meets their specific needs and expectations.

A second aspect of the present invention relates to a computer-implemented training method for training an epistemic Bayesian uncertainty model.

A first step comprises receiving training medical imaging data of training data through a first input interface of a data processing device. The respective sample of the training medical imaging data comprises voxels representing an anatomical region containing a training object set, which includes at least one training anatomical object. These data provide the basis for the training input data to train the epistemic Bayesian uncertainty model during the computer-implemented training method.

A next step comprises receiving training assignment information of the training data through a second input interface of the data processing device. The training assignment information describes the assignment of each voxel within the training medical imaging data to its respective training anatomical object. By incorporating this training assignment information as a training output, the epistemic Bayesian uncertainty model may learn the relationships between different anatomical structures comprising the anatomical objects and their corresponding voxels.

A next step comprises separating the training data into subsets according to a given specification, with each subset assigned to a respective base learner of a deep ensemble model of the epistemic Bayesian uncertainty model. This separation process allows for the parallelization and optimization of the learning process, as each base learner focuses on understanding and interpreting different aspects of the training data. In other words, each base learner is trained on a subset of the total samples rather than receiving access to the entire collection during the learning process. This partitioning approach enables individual models to develop independently, focusing on different aspects of the input data, which in turn fosters diversity among the base learners and enhances the overall performance and robustness of the deep ensemble model. By exposing each learner to a unique subset of samples, the system can better capture uncertainties and variations in the data, leading to improved generalization capabilities and more accurate predictions for out-of-distribution cases or novel situations.

After separating the training data into subsets, the base learners are trained based on their respective subsets using the computation unit of the data processing device. By providing individualized training experiences for each base learner, the epistemic Bayesian uncertainty model can develop a more comprehensive and nuanced understanding of the anatomical structures present within the training medical imaging data.

Finally, the epistemic Bayesian uncertainty model is provided through an output interface of the data processing device. The epistemic Bayesian uncertainty model may be provided to a data processing device to perform a method according to the first aspect of the present invention.

According to a further embodiment of the present invention, the computer-implemented method incorporates a specific data separation strategy when handling training data in the context of a request for diagnostic analysis specifying an object set. In this configuration, the training data is separated into eight subsets, with each subset assigned to a base learner in the deep ensemble.

The training data separation process adheres to strict guidelines designed to ensure even distribution and minimize redundancy between different subsets. Each base learner processes at most $N/2 + 1$ of the N cases from the total training data, with each case being included in exactly four of the eight subsets. This approach ensures that every base learner receives a unique yet overlapping subset of the training data, promoting more comprehensive learning and better generalization capabilities across various anatomical structures and medical imaging scenarios. Additionally, the overlap between any pair of subsets is limited to at most $N/4 + 1$ cases. This constraint further prevents overfitting and fosters robustness in the deep ensemble model by preventing individual base learners from becoming too specialized or reliant on specific patterns within their respective subsets. The use of this data separation strategy contributes to more accurate diagnostic outcomes by promoting diversity, comprehensiveness, and robustness within the computer-implemented method.

In general, the trained epistemic Bayesian uncertainty model and/or the trained contouring model mimics cognitive functions that humans associate with other human minds. In particular, by training based on training data the trained function is able to adapt to new circumstances and to detect and extrapolate patterns.

In general, parameters of a trained model can be adapted by means of training. In particular, supervised training, semi-supervised training, unsupervised training, reinforcement learning and/or active learning can be used. Furthermore, representation learning (an alternative term is "feature learning") can be used. In particular, the parameters of the trained functions can be adapted iteratively by several steps of training.

In particular, a trained model can comprise a neural network, a support vector machine, a decision tree and/or a Bayesian network, and/or the trained model can be based on k-means clustering, Q-learning, genetic algorithms and/or association rules. In particular, a neural network can be a deep neural network, a convolutional neural network or a convolutional deep neural network. Furthermore, a neural network can be an adversarial network, a deep adversarial network and/or a generative adversarial network.

According to a third aspect of the invention, a data processing device is provided, which is configured to perform a computer-implemented method according to the first aspect of the present invention and/or a method according to the second aspect of the present invention.

The data processing device may in particular be understood as a data processing device, which comprises processing circuitry. The data processing device can therefore in particular process data to perform computing operations. This may also include operations to perform indexed accesses to a data structure, for example a look-up table, LUT, as well as a data processing process implemented in hardware.

In particular, the data processing device may include one or more computers, one or more microcontrollers, and/or one or more integrated circuits, for example, one or more applicationspecific integrated circuits, ASIC, one or more field-programmable gate arrays, FPGA, and/or one or more systems on a chip, SoC. The data processing device may also include one or more processors, for example one or more microprocessors, one or more central processing units, CPU, one or more graphics processing units, GPU, and/or one or more signal processors, in particular one or more digital signal processors, DSP. The data processing device may also include a physical or a virtual cluster of computers or other of said units.

In various embodiments, the data processing device includes one or more hardware and/or software interfaces and/or one or more memory units.

A memory unit may be implemented as a volatile data memory, for example a dynamic random access memory, DRAM, or a static random access memory, SRAM, or as a non-volatile data memory, for example a read-only memory, ROM, a programmable read-only memory, PROM, an erasable programmable read-only memory, EPROM, an electrically erasable programmable read-only memory, EEPROM, a flash memory or flash EEPROM, a ferroelectric random access memory, FRAM, a magnetoresistive random access memory, MRAM, or a phase-change random access memory, PCRAM.

According to a fourth aspect of the invention, a medical imaging system is provided. The medical imaging system comprises a data processing device according to the third aspect of the present invention and a medical imaging device. The medical imaging device is configured to generate imaging raw data representing the anatomical region and to generate the medical imaging data depending on the imaging raw data.

The medical imaging device may, for example, be a CT device, a CBCT device, an MRI device or an X-ray device.

According to a fifth aspect of the present invention, a computer program product comprising instructions is provided. When the instructions are executed by a data processing device, the instructions cause the data processing device to carry out a computer-implemented method according to the first and/or the second aspect of the present invention. The instructions may be provided as program code, for example. The program code can for example be provided as binary code or assembler and/or as source code of a programming language, for example C, and/or as program script, for example Python.

According to a sixth aspect of the invention, a computer-readable storage medium, in particular a tangible and/or non-transient computer readable storage medium, storing a computer program according to the invention is provided. The computer program and the computer-readable storage medium are respective computer program products comprising instructions. The instructions cause the data processing device to carry out a computer-implemented method according to the first and/or the second aspect of the present invention.

Independent of the grammatical term usage, individuals with male, female or other gender identities are included within the term.

Further features and feature combinations of the invention are obtained from the figures and their description as well as the claims. In particular, further implementations of the invention may not necessarily contain all features of one of the claims. Further implementations of the invention may comprise features or combinations of features, which are not recited in the claims.

In the following, the invention will be explained in detail with reference to specific exemplary implementations and respective schematic drawings. In the drawings, identical or functionally identical elements may be denoted by the same reference signs. The description of identical or functionally identical elements is not necessarily repeated with respect to different figures.

In the Figures,

FIG 1     shows a schematic illustration of a medical imaging system;

FIG 2     shows a schematic illustration of an epistemic Bayesian uncertainty model;

FIG 3     shows a schematic illustration of a computer-implemented method;

FIG 4     shows a schematic illustration of a computer-implemented training method;

FIG 5     shows a schematic illustration of a post-processing;

FIG 6     shows a schematic illustration of a workflow for post-processing of an epistemic uncertainty map;

FIG 7     shows a schematic illustration of a contouring of an epistemic uncertainty map using a polygonisation procedure;

FIG 8     shows a schematic illustration of a mapping comprising epistemic uncertainty contours and anatomical object contours;

FIG 9     displays an embodiment of an artificial neural network. Alternative terms for "artificial neural network" are "neural network", "artificial neural net" or "neural net"; and

FIG 10    displays an embodiment of a convolutional neural network.

FIG 1 shows a schematic illustration of a medical imaging system. The medical imaging system 10 may comprise a data processing device 12, which may be configured to carry out a computer-implemented method to detect predefined anatomical objects 14 of an anatomical region 16. The medical imaging system 10 may also comprise a medical imaging device 18, which may for example be a CT-device or an MR-device. Optionally, the medical imaging system 10 comprises a patient table 20 or the like. The medical imaging device 18 is configured to generate medical imaging data 22 representing the anatomical region 16, of a body on the patient table 20. The medical imaging data 22 may comprise a three-dimensional representation of the anatomical region 16.

The medical imaging data 22 may comprise voxels. The anatomical region 16 may comprise an object set comprising at least one anatomical object 14. The medical imaging system 10 may comprise the data processing device 12. The data processing device 12 may comprise a request interface 24 configured to receive request information 26 provided by a user describing the object set. In other words, the request information 26 may describe the anatomical objects 14 of the object set. The anatomical objects 14 may comprise organs, bones, or implants located within the anatomical region 16. The request information 26 may comprise a request to provide a visualization 28 of a predefined region of interest of the anatomical region 16 based on the medical imaging data 22. The visualization 28 may visualize a 2-D plane in a cross section through the anatomical region 16. The visualization 28 may comprise an anatomical object contour 30 of the at least one anatomical object 14 enclosing a volume representing the anatomical object 14 in the region of interest. The volume of the at least one anatomical object 14 may be determined based on the voxels assigned to the respective anatomical object 14 and the region of interest.

The data processing device 12 may be configured to generate visualization 28 data comprising the visualization 28 of the predefined region, the anatomical object contour 30 of the at least one anatomical object 14 and at least one epistemic uncertainty information 52 34 and/or a scoring information 36.

The data processing device 12 may be configured to determine assignment information 54 describing the assignment of the respective voxels to the respective anatomical object 14. In other words, the medical imaging data 22 may comprise the voxels wherein some of the voxels represent the respective anatomical object 14.

The assignment information 54 of the respective voxels may be determined by the data processing device 12 by applying a predefined contouring model on the medical imaging data 22 by the computation unit. The assignment information 54 describe the assignment of the respective voxels to the anatomical object 14.

The data processing device 12 may be configured to determine for the at least one anatomical object 14 the respective anatomical object contour 30 enclosing the volume representing the anatomical object 14 in the region of interest.

However, it may be possible that there are uncertainties related to the assignment of the respective voxels to the respective anatomical objects 14.

The uncertainty may be based on epistemic uncertainty. Epistemic uncertainty may be based on an out-of-distribution case. The out-of-distribution case may occur when the anatomical region 16 represented in the medical imaging data 22 is not trained sufficiently during a training of the predefined contouring model.

In that situation unreliable result giving wrong contours of the predefined anatomical objects 14 may occur. It may be necessary to identify the out of distribution case.

Therefore the data processing device 12 may be configured to apply an epistemic Bayesian uncertainty model 38 on the medical imaging data 22. The epistemic Bayesian uncertainty model 38 may be configured to determine an epistemic uncertainty information 52 34, describing an epistemic uncertainty of an assignment information 54 describing an assignment of the respective voxel to the respective anatomical object 14. In other words, the epistemic Bayesian uncertainty model 38 is configured to determine for the respective voxels the epistemic uncertainty of the assignment information 54 which describes the assignment of the respective voxel to the respective anatomical object 14.

The out-of-distribution case may be identified by applying a scoring procedure on the epistemic uncertainty information 52 34, to determine a scoring information 36 indicating a presence of the out-of-distribution case. The scoring procedure may comprise a determination of the scoring information 36 related to the voxels assigned to the respective anatomical object 14, to the voxels assigned to the contour of the respective anatomical object 14 and/or to all of the voxels. The scoring information 36 may for example comprise an average of the assignment information 54 and/or a sudden information of the assignment information 54. The medical imaging system 10 may be configured to provide a warning signal by an output interface when the scoring information 36 satisfies a predefined out-of-distribution condition. The out-of-distribution condition may comprise a threshold value of the scoring information 36. The warning signal may be provided by an output interface of the data processing device 12. The warning signal may be directed to a display device of the medical imaging system 10 to show a visual warning on the display device.

The epistemic Bayesian uncertainty model 38 may comprise a deep ensemble or more model comprising base learners 40. Each of the base learners 40 is configured to determine a week assignment information 54 describing an assignment of the respective voxel to the respective anatomical object 14 in a respective forward pass. In other words, the deep ensemble model comprises the base learners 40 that may be trained on respective subsets of the training data. Therefore the base learners 40 may be trained on different subsets. The medical imaging data 22 may be provided to each of the base learners 40 to determine a respective week assignment information 54 describing the assignment of the respective voxel to the respective anatomical object 14 in a respective forward pass. In other words each of the base learners 40 is configured to provide the weak assignment of the respective voxels to the respective anatomical objects 14 in the respective forward pass.

Each of the base learners 40 comprises Monte Carlo droput layers 42 wherein the epistemic Bayesian uncertainty model 38 is configured to perform a respective Monte Carlo droput in the Monte Carlo droput layers 42 in the respective forward pass. In other words the medical imaging data 22 may be provided to at respective base learner 40 in respective forward passes. In each of the forward passes the Monte Carlo droput may be performed on the Monte Carlo droput layers 42 of the base learners 40. Therefore in each of the forward passes the Monte Carlo droput layers 42 may be different. Therefore each of the forward passes may provide a respective week assignment information 54 describing the assignment of the respective voxel to the respective anatomical object 14.

It may be possible that the deep ensemble model comprises thebase learners 40. It may be intended to perform four of the forward passes on each of the base learners 40 using Monte Carlo droput. Therefore by applying the deep ensemble model combined with Monte Carlo droput thirty two different week assignment information 54 describing the assignment of the respective voxel to the respective anatomical object 14 may be provided.

To determine the epistemic Bayesian uncertainty the data processing device 12 may determine the epistemic uncertainty information 52 34, of the respective voxel-based assignments of the weak assignment information 54 50 provided for the respective voxel in the respective forward passes by the base learners 40. In other words for each voxel the respective weak assignment information 54 50 of the different forward passes is provided the data processing device 12. The data processing device 12 may determine a variance of the weak assignment information 54 50 to determine the epistemic uncertainty information 52 34, for the respective voxel. The data processing device 12 may be configured to provide this epistemic uncertainty information 52 34 of at least one some of the voxels or of the voxels within the contour enclosing the volume representing the anatomical object 14 to a scoring procdure to provide a scoring information 36.

The data processing device 12 may be configured to perform a postprocessing procedure on the respective anatomical object 14, as a function of the epistemic uncertainty information 52 34, of at least some of the voxels of the respective anatomical object 14 and/or as a function of predefined morphological conditions. It may be possible that voxels at the boundary of the contour may be removed from the assignment to the respective object as a function of the respective epistemic uncertainty information 52 34. It may be possible that some voxels may be removed from the contour when they satisfy predefined morphological conditions.

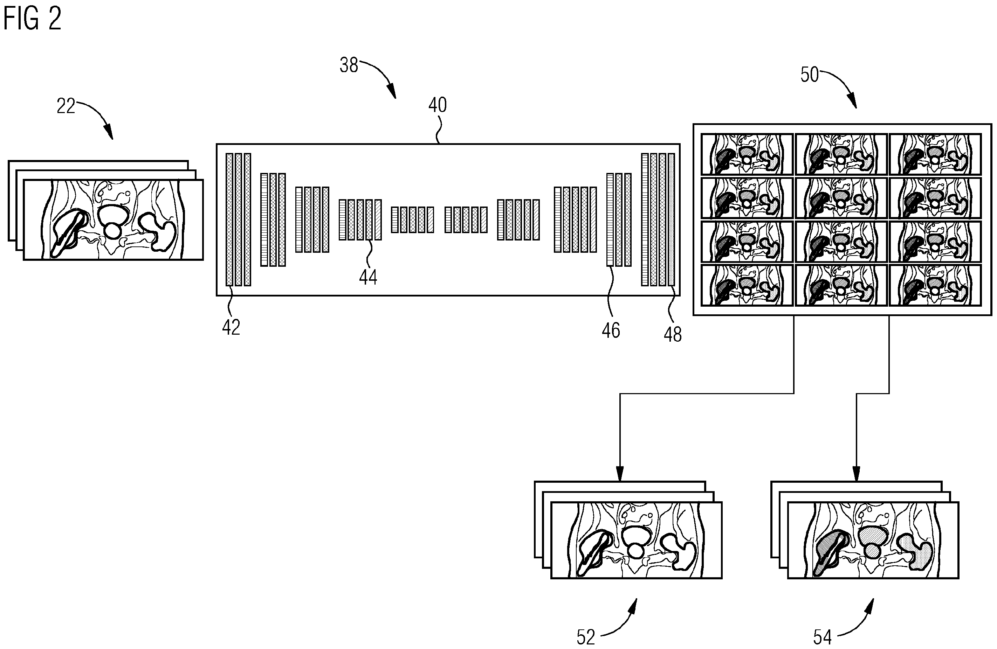

FIG 2 shows a schematic illustration of an epistemic Bayesian uncertainty model 38.

The epistemic Bayesian uncertainty model 38 may be applied to the medical imaging data 22. The medical imaging data 22 may be provided to base learners 40 of the epistemic Bayesian uncertainty model 38.

The base learners 40 within the epistemic Bayesian uncertainty model 38 are composed of layers 42 comprising convolutional (Conv) layers 42, batch normalization (BatchNorm) layers 42, and rectified linear unit (ReLU) activation functions, forming a foundation for processing medical imaging data 22. To account for epistemic uncertainty in the model's predictions, Monte Carlo dropout layers 44 42 are included after each ResBlock, enabling stochastic variability during training. This approach fosters an understanding of the model's confidence in its predictions and helps estimate the underlying uncertainty in the segmentation process. Additionally, the base learners 40 incorporate pooling layers 46 42 for downsampling spatial dimensions and/or upsampling layers 42 for restoring resolution when moving through the network hierarchy. The final layer 42 of each base learner 40 comprises a softmax function 48 to output weak assignment information 54 50 describing the assignment probability of each voxel belonging to a specific anatomical object 14. During each forward pass, Monte Carlo dropout is applied on the respective Monte Carlo dropout layers 44 42 to generate multiple hypotheses regarding the voxel-to-object assignments. This method introduces randomness into the model, enabling better quantification of epistemic uncertainty and facilitating more informed decision-making in the segmentation process. The input for each base learner 40 consists of medical imaging data 22 that undergo convolution, batch normalization, activation, pooling or upsampling, and softmax operations to produce weak assignment information 54 50, ultimately supporting a robust and adaptive approach to automated anatomical object 14 segmentation in medical imaging data 22.

The generation of epistemic uncertainty information 52 34 for each voxel within the medical imaging data 22 relies on the weak assignment information 54 50 generated by the base learners 40 in the epistemic Bayesian uncertainty model 38. During multiple forward passes, Monte Carlo dropout layers 44 42 introduce stochastic variability into the models, leading to different hypotheses regarding voxel-to-object assignments for each iteration. By collecting and analyzing these weak assignment information 54 50 outputs from several forward passes with varying randomness, the epistemic uncertainty associated with a specific voxel can be quantified. The degree of variation in the predicted probabilities among multiple runs serves as an indicator of the model's confidence in assigning the voxel to a particular anatomical object 14. Higher variability implies lower confidence and consequently higher epistemic uncertainty, while lower variability suggests greater certainty in the assignment. The process involves computing the entropy or variance of the weak assignment probabilities across various forward passes, resulting in a measure of epistemic uncertainty for each voxel. This metric reflects the model's confidence and enables users to better understand potential errors, inconsistencies, or ambiguities in the segmentation process.

The generation of assignment information 54, which describes the assignment of each respective voxel to a specific anatomical object 14, is derived from the weak assignment information 54 50 obtained from the base learners 40. During the process, base learners 40 produce multiple weak assignment hypotheses for each voxel through Monte Carlo dropout layers 44 42, resulting in probabilistic predictions about their affiliations with various anatomical objects 14. To convert these weak assignment information 54 50 into the assignment information 54, a decision rule is applied to consolidate the information from several forward passes. This can be achieved by selecting the highest probability anatomical object 14 for each voxel across all iterations as its final assignment. In cases where multiple anatomical objects 14 have similar probabilities, a threshold may be used to determine if a voxel should be assigned or marked as uncertain, reflecting ambiguity in the segmentation.

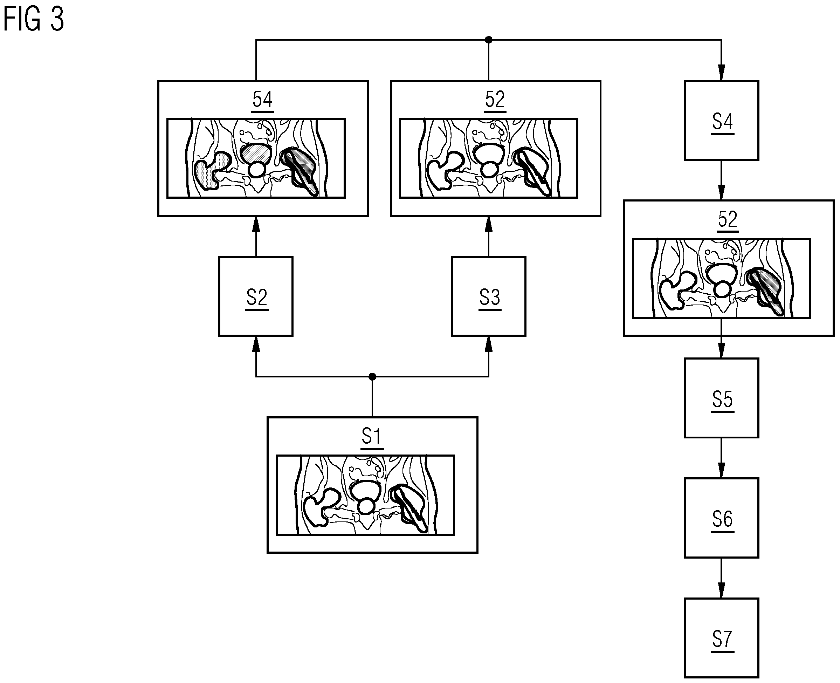

FIG 3 shows a schematic illustration of a computer-implemented method.

Step S1: The method begins with an input interface receiving medical imaging data 22 comprising voxels, which represent an anatomical region 16 consisting of at least one anatomical object 14 within an object set. These voxels are utilized to construct detailed images of internal body structures for further analysis and segmentation. Medical imaging data 22 can be derived from various modalities, such as Computed Tomography (CT), Magnetic Resonance Imaging (MRI), or Positron Emission Tomography (PET) scans.

Step S2: The computation unit applies a predefined contouring model on the medical imaging data 22 to establish assignment information 54 describing which voxels belong to the respective anatomical object 14. Following this, respective anatomical object contour 30s are generated based on these assigned voxels and the defined region of interest. The contouring model can be a machine learning algorithm or deep neural network designed for accurate segmentation of anatomical objects 14 in medical imaging data 22.

Step S3: In this step, the computation unit employs an epistemic Bayesian uncertainty model 38 on the medical imaging data 22 to assess epistemic uncertainty information 52 34, associated with the assignment information 54. Epistemic uncertainty stems from a lack of knowledge or understanding about the correct segmentation and can be quantified through statistical methods. This uncertainty information 52 helps determine how confident the contouring model is in assigning specific voxels to individual anatomical objects 14.

Step S4: Following the epistemic uncertainty assessment, post-processing of the respective anatomical object contour 30s takes place based on the epistemic uncertainty information 52 34, for some or all of the assigned voxels and predefined morphological conditions. Postprocessing can include various techniques such as smoothing, hole filling, and size filtering to ensure accurate and consistent contour representation.

Step S5: This step involves performing a scoring procedure that comprises determining a respective contour uncertainty score for each anatomical object contour 30 based on the epistemic uncertainty information 52 34 of voxels within the volume representing the respective anatomical object 14. Additionally, a total uncertainty score can be calculated based on the epistemic uncertainty information 52 34 for all anatomical objects 14 in the medical imaging data 22. This scoring procedure enables an overall assessment of segmentation quality and highlights areas where further improvement or manual intervention may be necessary.

Step S6: If the scoring information 36 meets a predefined out-of-distribution condition, a warning signal is provided to the user interface through an output interface. This alert informs users of potential issues with the segmentation results and encourages them to exercise caution when interpreting or using these outcomes.

Step S7: Finally, the user interface intuitively displays uncertainty information 52 by highlighting high-uncertainty areas and incorporating easy decision points such as a traffic light or single score indicating the trustworthiness of predicted segmentations. This visual representation helps users quickly assess segmentation quality, enabling informed decision-making and effective utilization of medical imaging data 22 for diagnostic or treatment planning purposes.

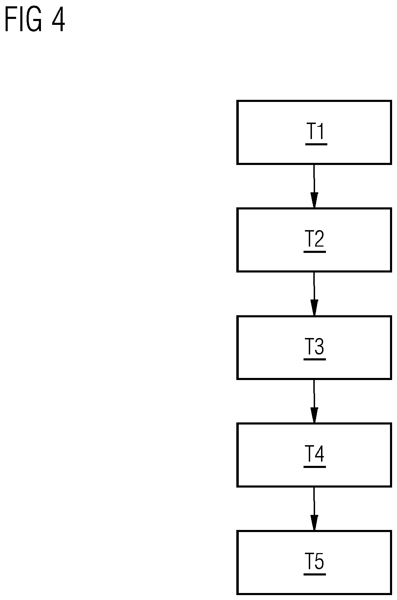

FIG 4 shows a schematic illustration of a computer-implemented training method.

Step T1: The computer-implemented training method begins with a first input interface receiving training medical imaging data 22 of training data, which consist of voxels representing an anatomical region 16 containing a training object set. These training data are used to instruct and optimize the epistemic Bayesian uncertainty model 38 for accurate segmentation. The training medical imaging data 22 can be derived from various modalities, such as Computed Tomography (CT), Magnetic Resonance Imaging (MRI), or Positron Emission Tomography (PET) scans.

Step T2: A second input interface on the data processing device 12 receives training assignment information 54 of the training data. This information describes which voxels in the training medical imaging data 22 belong to each training anatomical object 14, providing the ground truth needed for model training.

Step T3: The training data are separated into subsets based on a given specification. Each subset is then assigned to a respective base learner 40 of a deep ensemble model, which forms part of the epistemic Bayesian uncertainty model 38. This separation process increases the robustness and accuracy of the model by allowing each base learner 40 to specialize in different aspects or features of the training data.

Step T4: The computation unit of the data processing device 12 trains the base learners 40 using their respective subsets of training data. Training involves optimizing each base learner 40's performance to minimize the difference between its predictions and the ground truth training assignment information 54. This process is typically achieved through iterative techniques such as backpropagation and stochastic gradient descent.

Step T5: Once the base learners 40 have been trained, the epistemic Bayesian uncertainty model 38 is provided by an output interface of the data processing device 12. This completed model can then be used to assess segmentation uncertainties in new medical imaging data 22, providing valuable insights into the reliability and confidence of its predictions.

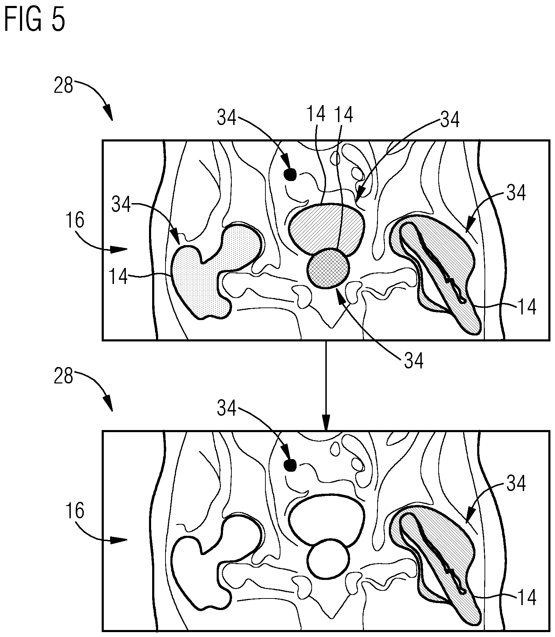

FIG 5 shows a schematic illustration of a post-processing.

FIG 5 shows a visualization 28 of an epistemic uncertainty map based of the epistemic uncertainty information 52 34 of the anatomical region 16. The upper visualization 28 of the anatomical region 16 shows the epistemic uncertainty map based on raw values of the epistemic uncertainty information 52 34 before the post-processing. A region around a hip implant shows higher epistemic uncertainties. These epistemic uncertainties may be due to the fact that no or an insufficient number of images with hip implants were provided in the training data. Other of the regions show high epistemic uncertainties at edges of the anatomical objects14. These edges may be due to boundary effects and not due to epistemic uncertainties. It may therefore be desirable to remove these edges.

The lower visualization 28 of the anatomical region 16 shows the epistemic uncertainty map based on after the post-processing. In this mapping, the edges are removed. However, the area around the hip implant is still present, as desired.

In general, the epistemic uncertainty maps can contain artifacts and boundary regions of high epistemic uncertainty, which may compromise contour generation's accuracy and reliability. To tackle this issue, morphological operations are employed during post-processing on epistemic uncertainty maps before contour extraction. These operations can remove voxels assigned to anatomical objects 14 or fill gaps within areas assigned to these objects.

Morphological dilation expands high-intensity region boundaries in epistemic uncertainty maps, incorporating neighboring pixels and filling gaps. This operation is useful for sparse or noisy data sets where small gaps may occur between voxels belonging to the same object.

Morphological erosion shrinks high-intensity regions by removing exterior voxels, eliminating small artifacts while preserving primary structures. This operation ensures a more accurate representation of anatomical objects 14 by removing extraneous signal noise or minor inconsistencies in the data.

Combined operations such as morphological opening (erosion followed by dilation) and closing (dilation followed by erosion) offer additional benefits in refining epistemic uncertainty maps. Opening removes small objects or bridges, smoothing contours without altering major structures, while closing fills gaps and connects disjointed regions for improved uniformity.

Morphological thinning reduces the thickness of elongated objects in epistemic uncertainty maps while preserving their topology. By identifying and removing extraneous voxels from anatomical objects 14, thinning improves contour accuracy without changing overall shape or structure, making it particularly useful for complex structures like blood vessels or neural networks.

Post-processing may be performed prior to the scoring procedure to determine contour uncertainty scores of respective contours in at least one anatomical object 14 based on epistemic uncertainty information 52 34 from voxels within the volume representing that object. The post-processing prior to the scoring procedure ensures that all regions containing voxels undergo the same pre-processing steps, resulting in consistent and standardised contour uncertainty scores. Removing voxels at a boundary can ensure that boundary effects on voxel mapping uncertainty can be filtered out.

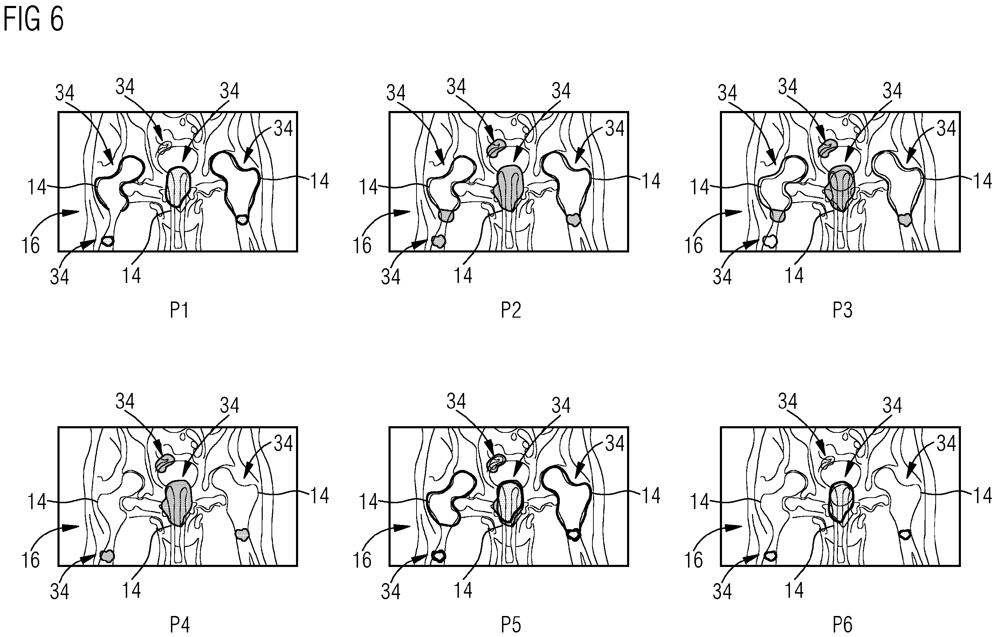

FIG 6 shows a schematic illustration of a workflow for post-processing of an epistemic uncertainty map.

The workflow for post-processing of an epistemic uncertainty map is initiated by receiving the input data, comprising epistemic uncertainty information 52 34 for each voxel in the volume representing an anatomical object 14 in a step P1.

In the first step P2, thresholding is applied to binarize the epistemic uncertainty map. A predefined threshold value is set, and all epistemic uncertainty information 52 34 below this level is set to 0, while all epistemic uncertainty information 52 34 equal to or above the threshold is set to 1. This process enables differentiation between high-uncertainty regions and low-uncertainty ones within the volume.

The second step P3 consists of performing a morphological dilation operation on the thresholded epistemic uncertainty map. By adding voxels to the boundaries of the connected components in the binary image, the size of high-uncertainty areas is increased, thereby ensuring that neighboring regions with high uncertainty values are included in the final analysis.

Following dilation, a morphological opening is performed in the third step P4. Opening combines erosion (shrinking object boundaries) and dilation (expanding object boundaries), effectively eliminating small unwanted regions (such as noise or outliers) from the binary image while preserving the overall shape of high-uncertainty areas within the volume.

The fourth step P5 involves applying a masking operation, where the epistemic uncertainty map generated in the morphological opening is used as a mask for the original input data. This process ensures that only voxels with high uncertainty values are included in the final result, while low-uncertainty regions are filtered out.

In the last step P6, the post-processed epistemic uncertainty map is displayed, showcasing refined information on high-uncertainty areas within the volume. The processed data can be utilized to determine scores for the respective anatomical object 14.

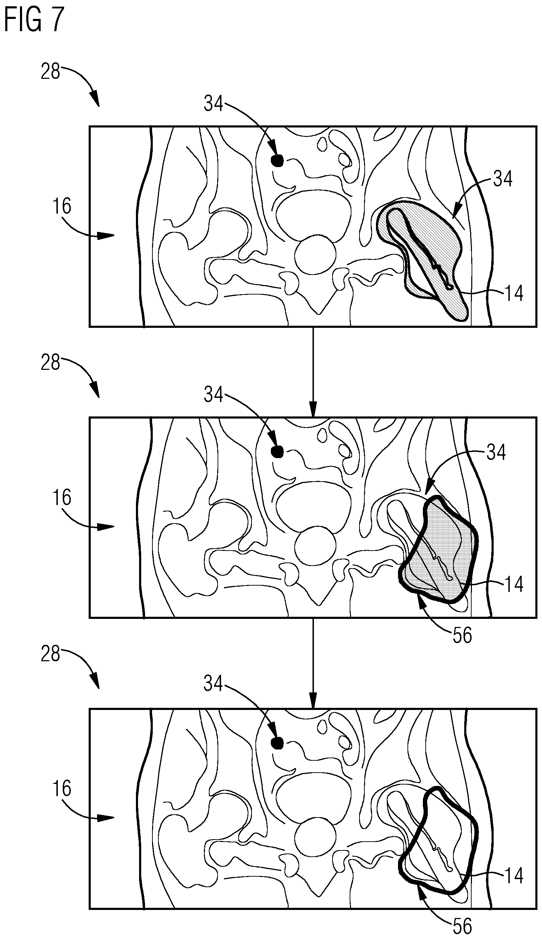

FIG 7 shows a schematic illustration of a contouring of an epistemic uncertainty map using a polygonisation procedure.

The epistemic uncertainty map may be generated in a post-processing procedure shown in FIG 6. A contouring procedure may be performed on the post-processed epistemic uncertainty map to generate a contour of epistemic uncertainty 56 around a region of epistemic uncertainty. The contour may be provided only for a region associated with a corresponding anatomical object 14.

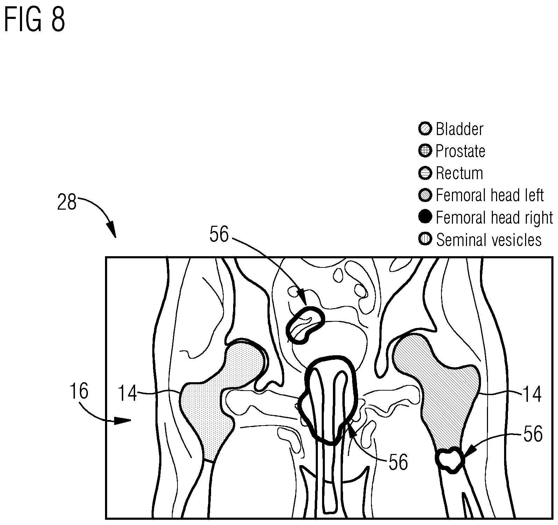

FIG 8 shows a schematic illustration of a mapping comprising contours of epistemic uncertainty 56 and anatomical object contour 30s.

A user may be provided with the option to perform a polygonisation procedure on areas of voxels assigned to their respective anatomical objects 14 during post-processing. This process converts areas of the voxels assigned to the anatomical object 14 into 2D mesh representations (polygons), allowing for easier visualization 28 and analysis of complex structures.

Polygonisation procedures include the Marching Cubes algorithm, which is widely used in scientific visualization 28 and computer graphics. The Marching Cubes algorithm generates a polygonal mesh from a set of voxels representing an object's boundary surface by examining each cell (cube) formed by eight neighboring voxels and creating triangles based on the voxel values within that cell.

Another example is the Surface Nets method, which generates a 2D mesh using spheres centered at each voxel location to approximate the surface of an object. This technique offers greater control over smoothing and preserving the shape of complex structures compared to Marching Cubes.

The Ball Pivoting algorithm is another polygonisation procedure that uses balls of varying radii to create a mesh from a set of voxels. By adjusting the ball radius, this method can handle varying levels of detail in the input data and provide more accurate representations of anatomical structures.

Performing the polygonisation procedure on areas after post-processing ensures that any noise, artifacts, or gaps within the assigned voxel regions are addressed before converting them into 2D meshes. This approach enhances the quality of visualization 28 and downstream analysis by producing cleaner, more accurate representations of anatomical structures in polygon form.

Using established methods such as Marching Cubes, Surface Nets, or Ball Pivoting algorithms, these procedures enable easier visualization 28 and analysis of complex structures while ensuring high-quality representations by addressing noise, artifacts, and gaps during postprocessing. The user may also perform these algorithms on the respective epistemic uncertainty map. The user may request a combination of the contours in a visualization 28 as shown in FIG 8

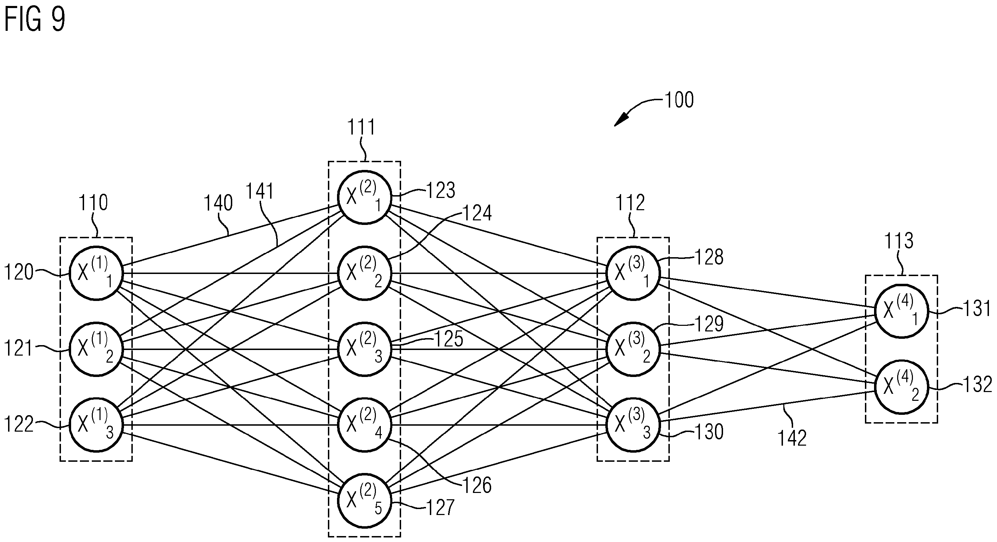

FIG 9 displays an embodiment of an artificial neural network. Alternative terms for "artificial neural network" are "neural network", "artificial neural net" or "neural net".

The trained function may comprise the artificial neural network 100.

The artificial neural network 100 comprises nodes 120, ..., 132 and edges 140, ..., 142, wherein each edge 140, ..., 142 is a directed connection from a first node 120, ..., 132 to a second node 120, ..., 132. In general, the first node 120, ..., 132 and the second node 120, ..., 132 are different nodes 120, ..., 132, it is also possible that the first node 120, ..., 132 and the second node 120, ..., 132 are identical. For example, in Fig. 1 the edge 140 is a directed connection from the node 120 to the node 123, and the edge 142 is a directed connection from the node 130 to the node 132. An edge 140, ..., 142 from a first node 120, ..., 132 to a second node 120, ..., 132 is also denoted as "ingoing edge" for the second node 120, ..., 132 and as "outgoing edge" for the first node 120, ..., 132.

In this embodiment, the nodes 120, ..., 132 of the artificial neural network 100 can be arranged in layers 110, ..., 113, wherein the layers can comprise an intrinsic order introduced by the edges 140, ..., 142 between the nodes 120, ..., 132. In particular, edges 140, ..., 142 can exist only between neighboring layers of nodes. In the displayed embodiment, there is an input layer 110 comprising only nodes 120, ..., 122 without an incoming edge, an output layer 113 comprising only nodes 131, 132 without outgoing edges, and hidden layers 111, 112 in-between the input layer 110 and the output layer 113. In general, the number of hidden layers 111, 112 can be chosen arbitrarily. The number of nodes 120, ..., 122 within the input layer 110 usually relates to the number of input values of the neural network, and the number of nodes 131, 132 within the output layer 113 usually relates to the number of output values of the neural network. In particular, a (real) number can be assigned as a value to every node 120, ..., 132 of the neural network 100. Here, $x^{(n)}_i$ denotes the value of the i-th node 120, ..., 132 of the n-th layer 110, ..., 113. The values of the nodes 120, ..., 122 of the input layer 110 are equivalent to the input values of the neural network 100, the values of the nodes 131, 132 of the output layer 113 are equivalent to the output value of the neural network 100. Furthermore, each edge 140, ..., 142 can comprise a weight being a real number, in particular, the weight is a real number within the interval [-1, 1] or within the interval [0, 1]. Here, $w^{(m,n)}_{i,j}$ denotes the weight of the edge between the i-th node 120, ..., 132 of the m-th layer 110, ..., 113 and the j-th node 120, ..., 132 of the n-th layer 110, ..., 113. Furthermore, the abbreviation $w^{(n)}_{i,j}$ is defined for the weight $w^{(n,n+1)}_{i,j}$.

In particular, to calculate the output values of the neural network 100, the input values are propagated through the neural network. In particular, the values of the nodes 120, ..., 132 of the (n+1)-th layer 110, ..., 113 can be calculated based on the values of the nodes 120, ..., 132 of the n-th layer 110, ..., 113 by

$$x^{(n+1)}_j = f\left(\sum_i x^{(n)}_i \cdot w^{(n)}_{i,j}\right)$$

Herein, the function f is a transfer function (another term is "activation function"). Known transfer functions are step functions, sigmoid function (e.g. the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network, wherein values of the input layer 110 are given by the input of the neural network 100, wherein values of the first hidden layer 111 can be calculated based on the values of the input layer 110 of the neural network, wherein values of the second hidden layer 112 can be calculated based in the values of the first hidden layer 111, etc.

In order to set the values $w^{(m,n)}_{i,j}$ for the edges, the neural network 100 has to be trained using training data. In particular, training data comprises training input data and training output data (denoted as $t_i$). For a training step, the neural network 100 is applied to the training input data to generate calculated output data. In particular, the training data and the calculated output data comprise a number of values, said number being equal with the number of nodes of the output layer.

In particular, a comparison between the calculated output data and the training data is used to recursively adapt the weights within the neural network 100 (backpropagation algorithm). In particular, the weights are changed according to

$$w'^{(n)}_{i,j} = w^{(n)}_{i,j} - \gamma \cdot \delta^{(n)}_j \cdot x^{(n)}_i$$

wherein $\gamma$ is a learning rate, and the numbers $\delta^{(n)}_j$ can be recursively calculated as

$$\delta^{(n)}_j = \left(\sum_k \delta^{(n+1)}_k \cdot w^{(n+1)}_{j,k}\right) \cdot f'\left(\sum_i x^{(n)}_i \cdot w^{(n)}_{i,j}\right)$$

based on $\delta^{(n+1)}_j$, if the (n+1)-th layer is not the output layer, and

$$\delta^{(n)}_j = \left(x^{(n+1)}_k - t^{(n+1)}_j\right) \cdot f'\left(\sum_i x^{(n)}_i \cdot w^{(n)}_{i,j}\right)$$

if the (n+1)-th layer is the output layer 113, wherein f' is the first derivative of the activation function, and $y^{(n+1)}_j$ is the comparison training value for the j-th node of the output layer 113.

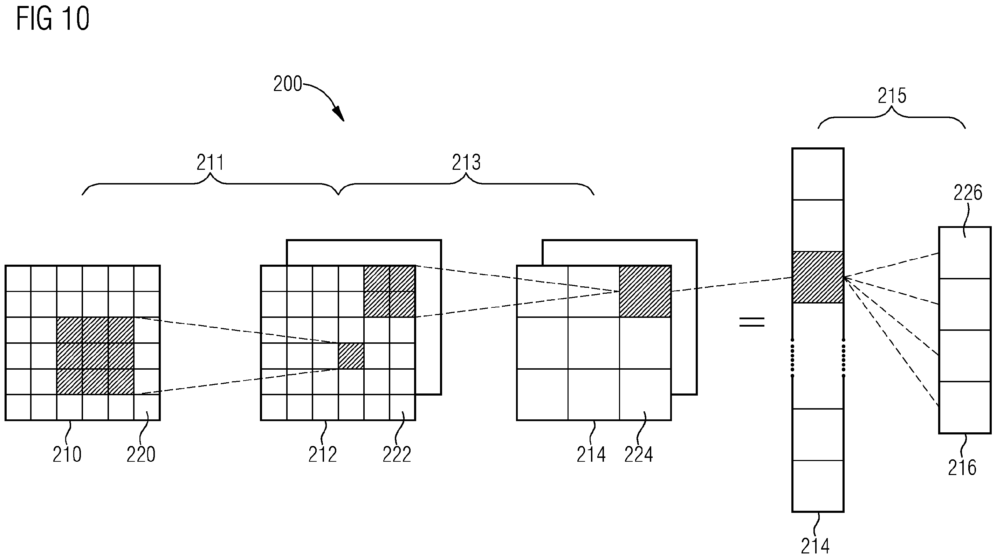

FIG 10 displays an embodiment of a convolutional neural network.

The trained function may comprise the convolutional neural network 200.

In the displayed embodiment, the convolutional neural network comprises 200 an input layer 210, a convolutional layer 211, a pooling layer 212, a fully connected layer 213 and an output layer 214. Alternatively, the convolutional neural network 200 can comprise several convolutional layers 211, several pooling layers 212 and several fully connected layers 213, as well as other types of layers. The order of the layers can be chosen arbitrarily, usually fully connected layers 213 are used as the last layers before the output layer 214.

In particular, within a convolutional neural network 200 the nodes 220, ..., 224 of one layer 210, ..., 214 can be considered to be arranged as a d-dimensional matrix or as a d-dimensional image. In particular, in the two-dimensional case the value of the node 220, ..., 224 indexed with i and j in the n-th layer 210, ..., 214 can be denoted as $x^{(n)}[i,j]$. However, the arrangement of the nodes 220, ..., 224 of one layer 210, ..., 214 does not have an effect on the calculations executed within the convolutional neural network 200 as such, since these are given solely by the structure and the weights of the edges.

In particular, a convolutional layer 211 is characterized by the structure and the weights of the incoming edges forming a convolution operation based on a certain number of kernels. In particular, the structure and the weights of the incoming edges are chosen such that the values $x^{(n)}_k$ of the nodes 221 of the convolutional layer 211 are calculated as a convolution $x^{(n)}_k = K_k * x^{(n-1)}$ based on the values $x^{(n-1)}$ of the nodes 220 of the preceding layer 210, where the convolution * is defined in the two-dimensional case as

$$x^{(n)}_k[i,j] = \left(K_k * x^{(n-1)}\right)[i,j] = \sum_{i'}\sum_{j'} K_k[i',j'] \cdot x^{(n-1)}[i-i', j-j']$$

Here the k-th kernel $K_k$ is a d-dimensional matrix (in this embodiment a two-dimensional matrix), which is usually small compared to the number of nodes 220, ..., 224 (e.g. a 3x3 matrix, or a 5x5 matrix). In particular, this implies that the weights of the incoming edges are not independent, but chosen such that they produce said convolution equation. In particular, for a kernel being a 3x3 matrix, there are only 9 independent weights (each entry of the kernel matrix corresponding to one independent weight), irrespectively of the number of nodes 220, ..., 224 in the respective layer 210, ..., 214. In particular, for a convolutional layer 211 the number of nodes 221 in the convolutional layer is equivalent to the number of nodes 220 in the preceding layer 210 multiplied with the number of kernels.

If the nodes 220 of the preceding layer 210 are arranged as a d-dimensional matrix, using a plurality of kernels can be interpreted as adding a further dimension (denoted as "depth" dimension), so that the nodes 221 of the convolutional layer 221 are arranged as a (d+1)-dimensional matrix. If the nodes 220 of the preceding layer 210 are already arranged as a (d+1)-dimensional matrix comprising a depth dimension, using a plurality of kernels can be interpreted as expanding along the depth dimension, so that the nodes 221 of the convolutional layer 221 are arranged also as a (d+1)-dimensional matrix, wherein the size of the (d+1)-dimensional matrix with respect to the depth dimension is by a factor of the number of kernels larger than in the preceding layer 210.

The advantage of using convolutional layers 211 is that spatially local correlation of the input data can exploited by enforcing a local connectivity pattern between nodes of adjacent layers, in particular by each node being connected to only a small region of the nodes of the preceding layer.

In the displayed embodiment, the input layer 210 comprises 36 nodes 220, arranged as a two-dimensional 6x6 matrix. The convolutional layer 211 comprises 72 nodes 221, arranged as two two-dimensional 6x6 matrices, each of the two matrices being the result of a convolution of the values of the input layer with a kernel. Equivalently, the nodes 221 of the convolutional layer 211 can be interpreted as arranges as a three-dimensional 6x6x2 matrix, wherein the last dimension is the depth dimension.

A pooling layer 212 can be characterized by the structure and the weights of the incoming edges and the activation function of its nodes 222 forming a pooling operation based on a nonlinear pooling function f. For example, in the two dimensional case the values $x^{(n)}$ of the nodes 222 of the pooling layer 212 can be calculated based on the values $x^{(n-1)}$ of the nodes 221 of the preceding layer 211 as

$$x^{(n)}[i,j] = f(x^{(n-1)}[id_1, jd_2], \ldots, x^{(n-1)}[id_1+d_1-1, jd_2+d_2-1])$$

In other words, by using a pooling layer 212 the number of nodes 221, 222 can be reduced, by replacing a number $d_1 \cdot d_2$ of neighboring nodes 221 in the preceding layer 211 with a single node 222 being calculated as a function of the values of said number of neighboring nodes in the pooling layer. In particular, the pooling function f can be the max-function, the average or the L2-Norm. In particular, for a pooling layer 212 the weights of the incoming edges are fixed and are not modified by training.

The advantage of using a pooling layer 212 is that the number of nodes 221, 222 and the number of parameters is reduced. This leads to the amount of computation in the network being reduced and to a control of overfitting.

In the displayed embodiment, the pooling layer 212 is a max-pooling, replacing four neighboring nodes with only one node, the value being the maximum of the values of the four neighboring nodes. The max-pooling is applied to each d-dimensional matrix of the previous layer; in this embodiment, the max-pooling is applied to each of the two two-dimensional matrices, reducing the number of nodes from 72 to 18.

A fully-connected layer 213 can be characterized by the fact that a majority, in particular, all edges between nodes 222 of the previous layer 212 and the nodes 223 of the fully-connected layer 213 are present, and wherein the weight of each of the edges can be adjusted individually.

In this embodiment, the nodes 222 of the preceding layer 212 of the fully-connected layer 213 are displayed both as two-dimensional matrices, and additionally as non-related nodes (indicated as a line of nodes, wherein the number of nodes was reduced for a better presentability). In this embodiment, the number of nodes 223 in the fully connected layer 213 is equal to the number of nodes 222 in the preceding layer 212. Alternatively, the number of nodes 222, 223 can differ.

Furthermore, in this embodiment the values of the nodes 224 of the output layer 214 are determined by applying the Softmax function onto the values of the nodes 223 of the preceding layer 213. By applying the Softmax function, the sum of the values of all nodes 224 of the output layer is 1, and all values of all nodes 224 of the output layer are real numbers between 0 and 1. In particular, if using the convolutional neural network 200 for categorizing input data, the values of the output layer can be interpreted as the probability of the input data falling into one of the different categories.

A convolutional neural network 200 can also comprise a ReLU (acronym for "rectified linear units") layer. In particular, the number of nodes and the structure of the nodes contained in a ReLU layer is equivalent to the number of nodes and the structure of the nodes contained in the preceding layer. In particular, the value of each node in the ReLU layer is calculated by applying a rectifying function to the value of the corresponding node of the preceding layer. Examples for rectifying functions are $f(x) = \max(0,x)$, the tangent hyperbolics function or the sigmoid function.

In particular, convolutional neural networks 200 can be trained based on the backpropagation algorithm. For preventing overfitting, methods of regularization can be used, e.g. dropout of nodes 220, ... , 224, stochastic pooling, use of artificial data, weight decay based on the L1 or the L2 norm, or max norm constraints.

## Claims

1. A computer-implemented method for detecting an out-of-distribution case, comprising:

   - receiving medical imaging data (22) comprising voxels, wherein the medical imaging data (22) represent an anatomical region (16) comprising an object set comprising at least one anatomical object (14), by an input interface of a data processing device (12);
   - applying an epistemic Bayesian uncertainty model (38) on the medical imaging data (22) by the computation unit to determine an epistemic uncertainty information (52) describing an epistemic uncertainty of an assignment information (54) describing an assignment of the respective voxel to the respective anatomical object (14),
   - applying a scoring procedure on the epistemic uncertainty information (52) to determine a scoring information (36);
   - provide a warning signal by an output interface if the scoring information (36) satisfies a predefined out-of-distribution condition;

   wherein

   - the epistemic Bayesian uncertainty model (38) comprises a deep ensemble model comprising base learners (40), wherein each of the base learners (40) is configured to determine a weak assignment information (50) describing an assignment of the respective voxel to the respective anatomical object (14) in a respective forward pass,
   - each of the base learners (40) is trained on a respective subset of training data,
   - each of the base learners (40) comprises Monte Carlo dropout layers (44), wherein the epistemic Bayesian uncertainty model (38) is configured to perform a respective Monte Carlo dropout in the Monte Carlo dropout layers (44) in the respective forward pass; and
   - the epistemic Bayesian uncertainty model (38) is configured to determine the epistemic uncertainty information (52) of the respective voxel based on a variance of the weak assignment information (50) provided for the respective voxel in the respective forward passes by the base learners (40).

2. The computer-implemented method according to claim 1, comprising:
   determining the assignment information (54) describing the assignment of the respective voxel based on a mean of the weak assignment information (50) provided for the respective voxel in the respective forward passes by the base learners (40).

3. The computer-implemented method according to claim 1, wherein the determining of the assignment information (54) comprises:

   - applying a predefined contouring model on the medical imaging data (22) by the computation unit to determine the assignment information (54) describing the assignment of the respective voxel to the anatomical object (14).

4. The computer-implemented method according to any one of the preceding claims, comprising:

   - generating a visualization (28) of a predefined region of interest of the anatomical region (16) based on the medical imaging data (22);
   - generating for the at least one anatomical object (14) a respective anatomical object contour (30) enclosing a volume representing the anatomical object (14) in the region of interest, based on the voxels assigned to the respective anatomical object (14) and the region of interest;
- generating review data comprising the visualization (28) of the predefined region, the anatomical object contour (30) of the at least one anatomical object (14) and at least one epistemic uncertainty information (34, 52) and/or the scoring information (36).

5.  The computer-implemented method according to any one of the preceding claims, comprising:

    - post-processing the respective anatomical object contour (30) as a function of the epistemic uncertainty information (52) of at least some of the voxels assigned to the respective anatomical object (14) and/or as a function of predefined morphological conditions.

6.  The computer-implemented method according to any one of the preceding claims, wherein:

    - the scoring procedure comprises a determination of a respective contour uncertainty score information of the respective anatomical object contour (30) of the respective at least one anatomical object (14) based on the epistemic uncertainty information (52) of the voxels within the volume representing the respective anatomical object (14) and/or a determination of a total uncertainty score information based on the epistemic uncertainty information (34, 52) of the voxels within the volumes representing the respective anatomical objects (14).

7.  The computer-implemented method according to any one of the preceding claims, wherein

    - the dropout layers (44) are arranged after every ResBlock layer of a respective base learner (40).

8.  The computer-implemented method according to any one of the preceding claims, comprising:

    - receiving a request information (26) describing the object set, by a request interface (24) of the data processing device (12).

9.  A computer-implemented training method for training an epistemic Bayesian uncertainty model (38), comprising:

    - receiving training medical imaging data (22) of training data, comprising voxels, wherein the training medical imaging data (22) represent an anatomical region (16) comprising a training object set comprising at least one training anatomical object (14), by a first input interface of a data processing device (12);
    - receiving training assignment information (54) of the training data describing an assignment of the respective voxels of the training medical imaging data (22) to the respective training anatomical object (14), by a second input interface of the data processing device (12);
    - separating the training data to subsets of the training data according to a given specification, wherein each of the subsets of the training data is assigned to a respective base learner (40) of a deep ensemble model of the epistemic Bayesian uncertainty model (38);
    - training the base learners (40) based on the respective subsets of the training data by the computation unit of the data processing device (12),
    - providing the epistemic Bayesian uncertainty model (38) by an output interface of the data processing device (12).

10. The computer-implemented training method according to claim 9, comprising:

    - training data is separated to eight of the subsets;
    - wherein the training data is separated such that

        each of the subsets base learner (40) is comprises at most $N/2 + 1$ of N cases of the training data, with every case being included in exactly four of the eight subsets;
        an overlap in samples between any pair of the subsets is limited to
        at most $N/4+1$ cases.

11. A data processing device (12) configured to carry out the method of one of the claims 1 to 8 or the method of one of the claims 9 to 10.

12. A medical imaging system (10) comprising an imaging device (18) and a data processing device (12) according to claim 11.

13. A computer program comprising instructions which, when the program is executed by a computer, cause the computer to carry out the method of one of the claims 1 to 8 or the method of one of the claims 9 to 10.

14. A computer-readable medium comprising instructions which, when executed by a computer, cause the computer to carry out the method of one of the claims 1 to 8 or the method of one of the claims 9 to 10.

FIG 1

FIG 2

FIG 3

FIG 4

```
┌─────────────────┐
│       T1        │
└─────────────────┘
         │
         ▼
┌─────────────────┐
│       T2        │
└─────────────────┘
         │
         ▼
┌─────────────────┐
│       T3        │
└─────────────────┘
         │
         ▼
┌─────────────────┐
│       T4        │
└─────────────────┘
         │
         ▼
┌─────────────────┐
│       T5        │
└─────────────────┘
```

# FIG 5

FIG 6

P1

P2

P3

P4

P5

P6

EP 4 715 757 A1

# FIG 7

FIG 8

⊘ Bladder
⊕ Prostate
⊖ Rectum
◐ Femoral head left
⬤ Femoral head right
◍ Seminal vesicles

FIG 9

FIG 10

EP 4 715 757 A1

**Europäisches Patentamt**
**European Patent Office**
**Office européen des brevets**

# EUROPEAN SEARCH REPORT

**Application Number**

EP 24 20 2134

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MOHAMMAD H JAFARI ET AL: "U-LanD: Uncertainty-Driven Video Landmark Detection", ARXIV.ORG, CORNELL UNIVERSITY LIBRARY, 201 OLIN LIBRARY CORNELL UNIVERSITY ITHACA, NY 14853, 2 February 2021 (2021-02-02), XP081871542, | 1-8,11, 13,14 | INV. G06V10/80 G06T7/00 |
| A | * page 1, column 1, line 1 - page 9, column 1, line 3; figures 1-5; tables 1-2 * | 9,10,12 | |
| A | KANNAN ARUNKUMAR ET AL: "Leveraging voxel-wise segmentation uncertainty to improve reliability in assessment of paediatric dysplasia of the hip", INTERNATIONAL JOURNAL OF COMPUTER ASSISTED RADIOLOGY AND SURGERY, SPRINGER, DE, vol. 16, no. 7, 9 May 2021 (2021-05-09), pages 1121-1129, XP037803009, ISSN: 1861-6410, DOI: 10.1007/S11548-021-02389-Y [retrieved on 2021-05-09] * page 1121, column 1, line 1 - page 1129, column 1, line 4; figures 1-4 * | 1-14 | |
| X | HAOCHEN MEI ET AL: "Automatic Segmentation of Gross Target Volume of Nasopharynx Cancer using Ensemble of Multiscale Deep Neural Networks with Spatial Attention", ARXIV.ORG, CORNELL UNIVERSITY LIBRARY, 201 OLIN LIBRARY CORNELL UNIVERSITY ITHACA, NY 14853, 27 January 2021 (2021-01-27), XP081868428, | 9-14 | |
| A | * page 1, column 1, line 1 - page 10, column 2, line 4; figures 1-12; tables 1-4 * | 1-8 | |

**TECHNICAL FIELDS SEARCHED (IPC)**

G06V
G06T

The present search report has been drawn up for all claims

| Place of search | Date of completion of the search | Examiner |
|---|---|---|
| The Hague | 5 February 2025 | Thibault, Guillaume |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 11275976 B2
- US 11185231 B2
- US 10600185 B2